(19) Europäisches Patentamt
European Patent Office
Office européen des brevets

(11) Veröffentlichungsnummer : **0 150 419**
**B1**

(12) # EUROPÄISCHE PATENTSCHRIFT

(45) Veröffentlichungstag der Patentschrift : **27.07.88**

(51) Int. Cl.[4] : **C 07 D471/04**, G 03 G 5/06 // (C07D471/04, 221:00, 249:00)

(21) Anmeldenummer : **84115643.3**

(22) Anmeldetag : **17.12.84**

(54) **Neue 2H-v-Triazolyl 4,5-b -pyridine und deren Verwendung.**

(30) Priorität : **13.01.84 DE 3400990**

(43) Veröffentlichungstag der Anmeldung : **07.08.85 Patentblatt 85/32**

(45) Bekanntmachung des Hinweises auf die Patenterteilung : **27.07.88 Patentblatt 88/30**

(84) Benannte Vertragsstaaten : **CH DE FR GB IT LI**

(56) Entgegenhaltungen :
**EP-A- 0 093 330**
**US-A- 3 058 989**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Albert, Bernhard, Dr.-Chem.**
**Eichenstrasse 60**
**D-6700 Ludwigshafen (DE)**
Erfinder : **Hoffmann, Gerhard, Dr.-Chem.**
**Pappelstrasse 22**
**D-6701 Otterstadt (DE)**
Erfinder : **Neumann, Peter, Dr.-Chem.**
**Franz-Schubert-Strasse 1**
**D-6908 Wiesloch (DE)**

EP 0 150 419 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue 2H-v-Triazolyl [4,5-b]-pyridine und deren Verwendung als Ladungsträger transportierende Verbindungen in elektrophotographischen Aufzeichnungsmaterialien.

Elektrophotographische Verfahren, dafür benötigte Materialien und verschiedene Varianten für den Aufbau von Aufzeichnungsmaterialien sind bekannt. Vorteilhaft für den Einsatz im Reproduktionssektor sind Materialien aus polymeren Bindemitteln, die an die speziellen Anforderungen des jeweiligen Einsatzgebietes angepaßt werden können, aus niedermolekularen organischen Verbindungen, die in den Bindemitteln auch in höheren Konzentrationen löslich und zu einem Transport von Ladungsträgern des elektrischen Stromes befähigt sind, sowie Verbindungen, insbesondere Farbstoffe oder Pigmente, die durch Absorption des bildmäßig eingestrahlten, aktinischen Lichtes Ladungsträger des elektrischen Stromes erzeugen und diese unter Mithilfe des von außen durch die elektrostatische Oberflächenladung aufgeprägten elektrischen Feldes auf die Ladung transportierenden Verbindungen übertragen können. Diese Ladungsträger erzeugenden Verbindungen können je nach Einsatzgebiet des Aufzeichnungsmaterials als eigene Schicht innerhalb einer Kompositstruktur eingebracht werden (DE-A 22 20 408) oder in Form monodispers gelöster Farbstoffmoleküle in der Mischung aus Bindemittel und Ladungsträger transportierenden Verbindungen vorhanden sein (DE-C 10 58 836). Das in der DE-A- 22 20 408 beschriebene mehrlagige elektrophotographische Aufzeichnungsmaterial besteht aus einem elektrisch leitfähigen Trägermaterial, einer ersten, Farbstoff enthaltenden, etwa 0,005 bis 2 µm dicken, durch Belichtung mit aktinischen Licht Ladungsträger des elektrischen Stromes erzeugenden Schicht aus im Dunkeln isolierenden, organischen Materialien mit mindestens einer Ladungen transportierenden Verbindung.

Aus der EP-A-093 330 sind elektrophotographische Aufzeichnungsmaterialien bekannt, die als Ladungsträger transportierende Verbindungen Triazole der Formel

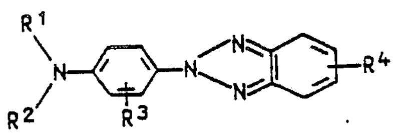

enthalten, in $R^1$ und $R^2$ u. a. Wasserstoff, Alkyl, Allyl, Benzyl oder gegebenenfalls substituiertes Phenyl,

$R^3$ Wasserstoff, Alkyl, Alkoxy oder Halogen und

$R^4$ Wasserstoff, Alkyl, Alkoxy, Vinyl, Allyl, Dialkylamino, Nitro, Cyan oder Acryloyl bedeuten.

Es ist auch bekannt, photohalbleitende organische Verbindungen zur Herstellung von elektrophotographischen Druckformen und insbesondere elektrophotographischen Offsetdruckformen zu verwenden (vgl. DE-C- 11 17 391 und 11 20 875, DE-B 12 55 497 und 27 26 116).

Die gestiegenen Anforderungen an Reproduktionssysteme verlangen eine Vielzahl von Aufzeichnungsmaterialien und -systemen, um für spezielle Probleme optimale Lösungen aussuchen zu können. Gewünscht ist eine hohe Lichtempfindlichkeit, eine gute Auflösung und gute Betonerung. Die oft beanstandete ungenügende Betonerung, die auf eine ungünstige Feldstärkedifferenzierung zwischen belichteten und unbelichteten Flächen hinweist, ist hierbei oft auf eine zu hohe Dunkelleitfähigkeit des Aufzeichnungsmaterials im beladenen Zustand zurückzuführen, so daß eine ungenügende Oberflächenladungsdichte vor der bildmäßigen Belichtung mit aktinischem Licht vorliegt.

Der vorliegenden Erfindung lag die Aufgabe zugrunde, neue Ladungsträger transportierende Verbindungen für elektrophotographische Aufzeichnungsmaterialien, insbesondere für die Herstellung von elektrophotographischen Druckformen wie Offsetdruckformen zu entwickeln, die bei hoher Lichtempfindlichkeit, guter Auflösung und Verarbeitung gleichzeitig ein geringes Dunkelleitvermögen aufweisen.

Es wurde nun gefunden, daß man verbesserte elektrophotographische Aufzeichnungsmaterialien mit elektrisch leitenden Trägern, Ladungsträgern erzeugenden Verbindungen bzw. Sensibilisatoren und Ladungsträger transportierenden Verbindungen erhält, wenn diese Materialien als Ladungsträger transportierende Verbindungen neue 2H-v-Triazolyl [4,5-b]-pyridine der Formel (I) enthalten

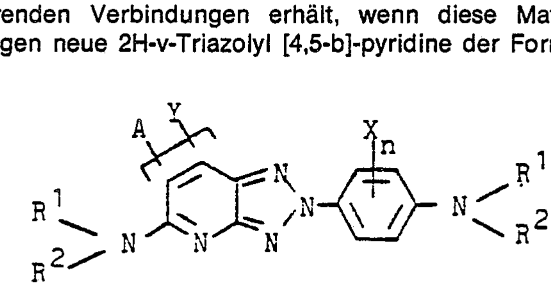

(I)

in der

$R^1$ und $R^2$ unabhängig voneinander für $C_1$- bis $C_4$-Alkyl, $C_5$- bis $C_7$-Cycloalkyl, für gegebenenfalls durch Chlor, Brom, $C_1$- bis $C_4$-Alkyl und/oder $C_1$- bis $C_4$-Alkoxy substituiertes Phenyl, Phenalkyl mit

insgesamt 7 bis 10 C-Atomen oder

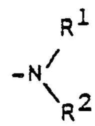

für einen gesättigten 5- oder 6-gliedrigen heterocyclischen Ring, und wobei die beiden

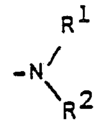

-Gruppen gleich oder veschieden sein können,

X für Wasserstoff, $C_1$- bis $C_4$-Alkyl, Halogen oder Phenyl,

n für 1 oder 2,

A für Wasserstoff, Halogen, Cyan, $C_1$- bis $C_4$-Alkyl, für gegebenenfalls durch Chlor, Brom und/oder $C_1$- bis $C_4$-Alkyl substituiertes Phenyl, Phenalkyl mit insgesamt 7 bis 10 C-Atomen, $C_1$- bis $C_4$-Alkoxy, für gegebenenfalls durch Chlor, Brom und/oder $C_1$- bis $C_4$-Alkyl substituiertes Phenoxy, Phenalkoxy mit insgesamt 7 bis 10 C-Atomen oder für

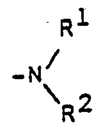

worin $R^1$ und $R^2$ oder

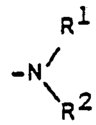

die oben angegeßene Bedeutung haben, und $R^1$ auch Wasserstoff sein kann, und

Y für Wasserstoff, Halogen oder $C_1$- bis $C_4$-Alkyl stehen.

Die elektrophotographischen Aufzeichnungsmaterialien, welche Triazolylpyridine gemäß der vorliegenden Erfindung enthalten, zeichnen sich durch eine Kombination sehr guter Eigenschaften, insbesondere einer hohen Photoleitfähigkeit bei gleichzeitig sehr niedriger Dunkelleitfähigkeit aus, so daß die Schichten für die Kopiertechnik sehr geeignet sind. Deutliche Vorteile weisen sie bei der Verwendung für die Herstellung von elektrophotographischen Druckformen auf und genügen hierbei hohen Ansprüchen im Hinblick auf das Auflösungsvermögen und die Druckauflage.

Als Substituenten $R^1$ und $R^2$ kommen für I z. B. im einzelnen in Betracht :

$C_1$- bis $C_4$-Alkyl : Methyl, Ethyl, n- und i-Propyl, n-Butyl, Isobutyl ; Cycloalkyl : Cyclopentyl, Cyclohexyl, Cycloheptyl ; gegebenenfalls substituiertes Phenyl : Phenyl, 2-, 3- und 4-Tolyl, 2-, 3- und 4-Methoxyphenyl, 2-, 3- und 4-Ethoxyphenyl, 2-, 3- und 4-Chlorophenyl, 2-, 3- und 4-Bromphenyl, 3- und 4-Ethylphenyl, 3- und 4-Isopropylphenyl, 3- und 4-sec.-Butylphenyl ; Phenalkyl : Benzyl, 2- und 1-Ethylphenyl, 2- und 3-Propylphenyl, Butylphenyl.

Als gesättigte 5- und 6-gliedrige heterocyclische Ringe sind für

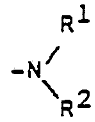

z. B. zu nennen : Reste des Pyrrolidins, Piperidins, Morpholins und N-$C_1$- bis $C_4$-Alkylpiperazins, wie N'-Methyl-, N'-Ethyl-, N'-Butylpiperazin.

Für $R^1$ und $R^2$ sind Methyl, Ethyl, Phenyl und Benzyl und für

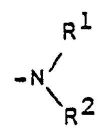

als heterocyclischer Rest Morpholinyl, Piperidinyl, Pyrrolidinyl und N'-$C_1$- bis $C_4$-Alkylpiperazinyl bevorzugt.

Als Substituenten X sind außer Wasserstoff und Phenyl im einzelnen z. B. zu nennen : als $C_1$- bis $C_4$-Alkyl : Methyl, Ethyl, Propyl und n- und i-Butyl, als Halogen : Brom, Fluor und insbesondere Chlor, wobei n 1 oder 2 ist.

Vorzugsweise steht X für Wasserstoff.

Für A kommen in Betracht :

1) Wasserstoff, Halogen, z. B. Brom, Fluor und Chlor und Cyan ;

2) $C_1$- bis $C_4$-Alkyl : Methyl, Ethyl, i- und n-Propyl, n-Butyl und Isobutyl ;

3) $C_1$- bis $C_4$-Alkoxy : Methoxy, Ethoxy, i- und n-Propoxy, n-Butoxy und Isobutoxy ;

4) gegebenenfalls substituiertes Phenyl : Phenyl, 2-, 3- und 4-Tolyl, 2-, 3- und 4-Chlorphenyl, 2-, 3- und n-Bromphenyl, 2-, 3- und n-Methoxyphenyl ;

5) gegebenenfalls substituiertes Phenoxy : Phenoxy, 2-, 3- und 4-Tolyloxy, 3- und 4-Chlorphenoxy ; 3- und 4-Bromphenoxy, 2-, 3- und 4-Methoxyphenoxy ;

6) Phenalkyl : Benzyl, 1- und 2-Ethylphenyl und 2- und 3-Propylphenyl ;

7) Phenalkoxy : Benzyloxy, 2-Phenylethoxy ;

8)

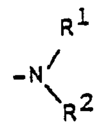

wobei $R^1$ und $R^2$ oder

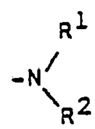

die oben angegebene Bedeutung haben.

Y steht in der Formel I für Wasserstoff, Halogen, z. B. Chlor, Brom oder Fluor, oder für $C_1$- bis $C_4$-Alkyl, z. B. Methyl, Ethyl, i- und n-Propyl sowie n- und i-Butyl.

Vorzugsweise ist A Wasserstoff, Chlor, Cyan oder Methyl.

Y steht vorzugsweise für Methyl oder Wasserstoff.

Wegen der hervorragenden anwendungstechnischen Eigenschaften sind Triazolylpyridine der Formel (II)

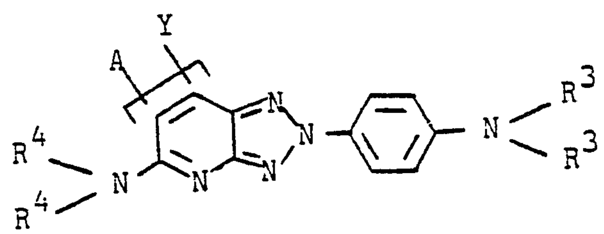

(II)

in der

$R^3$ und $R^4$ unabhängig voneinander für Methyl, Ethyl, Benzyl oder Phenyl,

A für Wasserstoff, Cyan, Chlor oder Methyl und

Y für Wasserstoff oder Methyl stehen, besonders hervorzuheben.

Besonders bevorzugt sind Verbindungen der Formel II, in der A und Y für Wasserstoff stehen.

Von diesen Verbindungen sind solche der Formel (III) ganz besonders hervorzuheben :

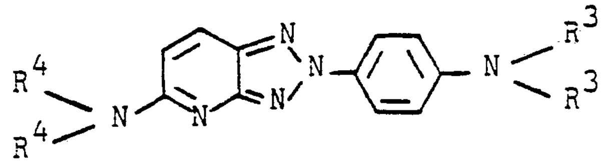

(III)

worin $R^3$ und $R^4$ die folgende Bedeutung haben :

| Verbindung | $R^3$ | $R^4$ |
|---|---|---|
| IIIa | $-CH_2-$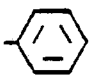 | $-C_2H_5$ |
| IIIb | $-C_2H_5$ | $-C_2H_5$ |
| IIIc | $-C_2H_5$ | $-CH_2-$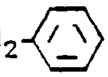 |

Die neuen Triazolyl [4,5-b] pyridine (I) können nach an sich bekannten Verfahren hergestellt werden. So können Verbindungen (I), in denen $R^1$ und $R^2$ aliphatische, cycloaliphatische oder phenylaliphatische Reste sind, beispielsweise auf folgendem Weg erhalten werden :

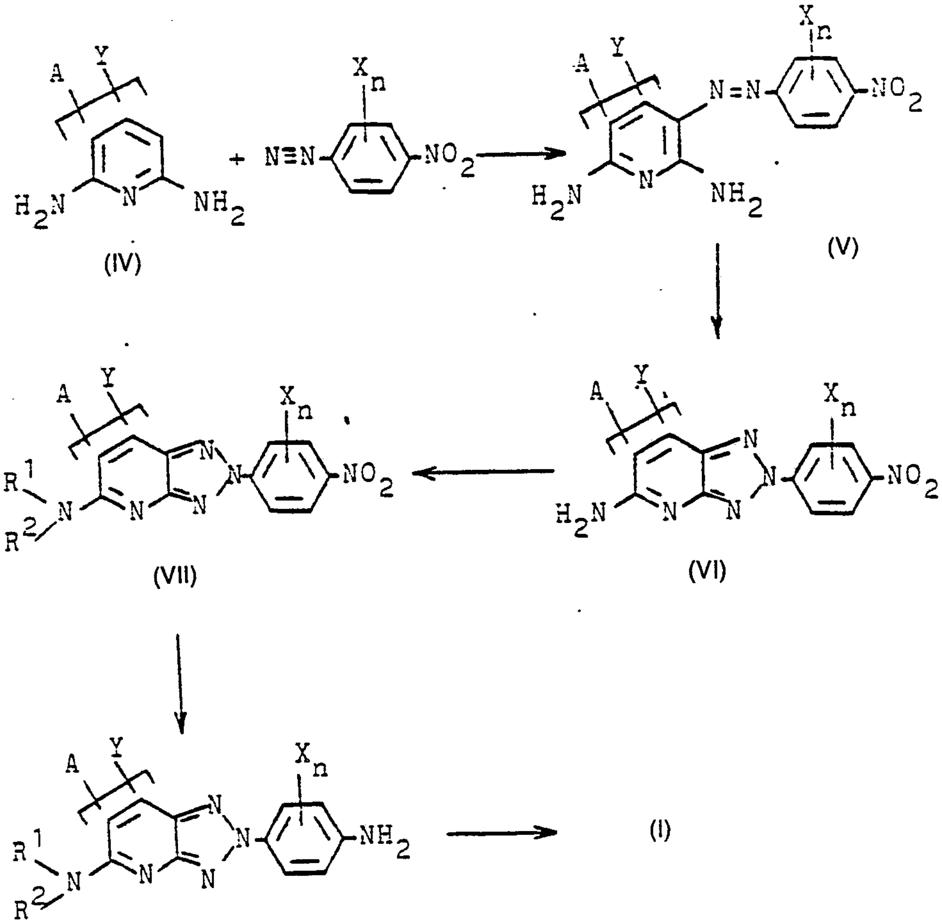

(IV) (V) (VI) (VII) (VIII) (I)

In den Formeln haben A, Y, X und n die oben angegebenen Bedeutungen. $R^1$ und $R^2$ stehen für die genannten aliphatischen, phenylaliphatischen und/oder cycloaliphatischen Reste.

Die Kupplung auf (IV) erfolgt in der Regel bei Temperaturen von — 10 bis + 20 °C.

Für die Oxidation von (V) zu (VI) kommen als Oxidationsmittel die verschiedensten in Betracht, z. B. Chromsäure, Hypochlorit, Alkalidichromate, Wasserstoffperoxid, Bleitetraacetat, Kaliumferricyanid, Ferrichlorid und Kupfer(II)-sulfat. In sauren Lösungsmitteln, z. B. wäßriger Essigsäure, werden vorzugsweise Alkalidichromate, Wasserstoffperoxid oder Bleitetraacetat und in basischen Lösungsmitteln, z. B. Pyridin-Wassergemisch, vorzugsweise Kaliumferricyanid, verwendet. Der oxidative Ringschluß erfolgt vorzugsweise mit Kupfer-(II)-sulfat in einem Pyridin-Wassergemisch oder in Tetrahydrofuran-Wassergemischen mit Hypochlorit. Die Oxidation mit Kupfer-(II)-salzen, wie Kupfer-(II)-sulfat oder -chlorid läßt sich

mit Vorteil auch in Methanol oder Methanol-Wassergemischen in Gegenwart von Ammonium- oder Aminsalzen, wie Mono- oder Dialkanolaminen durchführen. Der oxidative Ringschluß wird bei einer Temperatur von 70 bis 100 °C, vorzugsweise 90 bis 100 °C, durchgeführt.

Die Herstellung der Triazolylpyridine der Formel (I) wird im einzelnen durch die Ausführungsbeispiele erläutert.

Die neuen Triazolpyridine der Formeln (I), (II) und (III) sind hervorragend als Ladungsträger transportierende Verbindungen in elektrophotographischen Schichten geeignet. Die neuen Verbindungen können mit Vorteil sowohl in einschichtigen als auch in mehrschichtigen auf elektroleitfähige Träger aufgebrachten Aufzeichnungssystemen verwendet werden.

Für diesen Verwendungszweck haben sich die Verbindungen der Formel (II) und insbesondere die Verbindungen der Formel (III) bewährt. Von den letzteren sind die Verbindungen der Formeln (IIIa), (IIIb) und (IIIc) ganz besonders hervorzuheben.

Geeignete einschichtige Systeme weisen bevorzugt auf einem leitfähigen Trägermaterial eine Schicht auf, die aus (a) 45 bis 75 Gewichtsteilen eines Bindemittels, (b) 30 bis 60, insbesondere 35 bis 50 Gewichtsteilen der erfindungsgemäßen Ladungsträger transportierenden Verbindungen I, (c) gegebenenfalls bis 25 Gewichtsteilen eines weiteren, im wesentlichen inaktiven Bindemittels und (d) 0,05 bis 0,8 Gewichtsteilen einer bei aktinischer Belichtung Ladungsträger erzeugenden Verbindung, insbesondere eines geeigneten Farbstoffs besteht. Die Schichten werden mit Vorteil aus einer ca. 6 gew.%igen Lösung in einem geeigneten organischen Lösungsmittel auf das gereinigte leitfähige Trägermaterial so aufgebracht, daß nach dem Ablüften des Lösungsmittels je nach Verwendungszweck eine Trockenschichtdicke von ca. 0,8 bis 40 µm, bei elektrophotographischen Druckformen insbesondere 0,8 bis 6 µm, resultiert.

Geeignete Mehrschichtsysteme haben auf einem elektroleitfähigen Trägermaterial z. B. (a) eine Ladungsträger erzeugende Schicht und (b) eine Ladungstransportschicht aus 30 bis 60 Gewichtsteilen mindestens einer Ladungsträger transportierenden Verbindung der Formel I, 45 bis 75 Gewichtsteilen eines organischen Bindemittels und gegebenenfalls bis 25 Gewichtsteilen weiterer, die mechanischen Eigenschaften der Schicht verbessernde Zusätze. Die erste Schicht wird vorteilhaft in einer Dicke von 0,005 bis 5 µm, insbesondere 0,1 bis 0,9 µm als Lösung in einem geeigneten Lösungsmittel auf das Trägermaterial aufgetragen. Nach dem Auftrag erfolgt der Auftrag der zweiten Schicht in einer Dicke, daß nach dem Trocknen der Kompositstruktur eine Schichtdicke von 5 bis 25, insbesondere 7 bis 15 µm resultiert.

Als elektrisch leitende Träger sind prinzipiell alle leitfähigen Trägermaterialien verwendbar, soweit sie für die vorgesehene Anwendung geeignet sind. Bevorzugt sind je nach dem Anwendungsgebiet der Aufzeichnungsmaterialien Aluminium-, Zink-, Magnesium-, Kupfer- oder Mehrmetallplatten, z. B. rohe oder vorbehandelte, z. B. aufgeraute und/oder anodisierte Aluminiumbleche, Aluminiumfolien, Polymerfilme mit metallisierter Oberfläche wie aluminiumbedampfte Polyethylentetraphthalatfilme oder auch elektrisch leitende Spezialpapiere. Träger für Druckformen haben vorteilhaft eine Dicke von 0,08 bis ca. 0,3 mm.

Die Art der geeigneten organischen Bindemittel für die Schichten richtet sich nach dem beabsichtigten Verwendungszweck der Aufzeichnungsmaterialien. Für den Kopiersektor eignen sich. z. B. Celluloseether, Polyesterharze, Polyvinylchloride, Polycarbonate, Copolymers, wie Styrol-Maleinsäureanhydrid-Copolymere oder Vinylchlorid-Maleinsäureanhydrid-Copolymere oder Mischungen solcher Bindemittel. Bei ihrer Auswahl spielen ihre filmbildenden und elektrischen Eigenschaften, ihre Haftfestigkeit auf dem Trägermaterial und ihre Löslichkeitseigenschaften eine besondere Rolle. Insbesondere bei Aufzeichnungsmaterialien für die Herstellung elektrophotographischer Druckplatten und besonders bei denen für den Offsetdruck sind solche besonders geeignet, die in basischen, wäßrigen oder alkoholischen Lösungsmitteln löslich sind. Dies sind vor allem Substanzen mit alkalilöslich machenden Gruppen wie Anhydrid, Carboxyl-, Sulfonsäure-, Phenol- oder Sulfonimid-Gruppierungen. Bevorzugt sind Bindemittel, insbesondere solche mit hohen Säurenzahlen, die in basischen wäßrig-alkoholischen Lösungsmittelsystemen leicht löslich sind und ein mittleres Molekulargewicht (Gewichtsmittel) von 800 bis 80 000 und insbesondere 1 500 bis 50 000 aufweisen. Geeignet sind z. B. Copolymerisate aus Methacrylsäure und Methacrylsäureestern, besonders Copolymerisate aus Styrol und Maleinsäureanhydrid und aus Styrol, Methacrylsäure und Methacrylsäureester, soweit sie die vorstehende Löslichkeitsbedingung aufweisen. Obwohl bekanntermaßen Bindemittel mit freien Carboxylgruppen die Dunkelleitfähigkeit der elektrophotographischen Schichten in unerwünschter Weise erhöhen und dadurch zu schlechten Betonerungsergebnissen führen, lassen sich solche Bindemittel leicht an die erfindungsgemäß verwendeten Phenylindazole anpassen. So hat sich gezeigt, daß Copolymerisate aus Styrol, Maleinsäureanhydrid und Acryl- oder Methacrylsäure, die einen Anteil von einpolymerisiertem Maleinsäureanhydrid von 5 bis 50 Gew.% und einen Anteil von einpolymerisierter Acryl- oder Methacrylsäure von 5 bis 35, insbesondere 10 bis 30 Gew.% aufweisen, befriedigende elektrophotographische Schichten mit hinreichender Dunkelleitfähigkeit ergeben. Sie weisen eine hervorragende Löslichkeit in Auswaschmitteln aus 75 Gew.% Wasser, 23 Gew.% Isobutanol und 2 Gew.% Soda auf, sind aber in offsettypischem Wischwasser unlöslich.

Geeignete Ladungsträger erzeugende Verbindungen bzw. Sensibilisatoren sind z. B. für einschichtig aufgetragene Systeme, wie sie auch zur Herstellung elektrophotographischer Druckformen dienen, Farbstoffe aus der Triarylmethanreihe, Xanthenfarbstoffe und Cyaninfarbstoffe. Sehr gute Ergebnisse

wurden mit den erfindungsgemäßen Verbindungen der Formel I und Rhodamin B (C.I. 45170), Rhodamin 6 G (C.I. 45160), Malachitgrün (C.I. Basic Green 4 ; C.I. 4200), Methylviolett (C.I. 42535) oder Kristallviolett (C.I. 42555) erhalten. Bei mehrschichtig aufgetragenen Systemen liegt der Farbstoff oder das Pigment in einer separaten Ladungsträger erzeugenden Schicht vor. Hier sind Azofarbstoffe, Phthalocyanine, Isoindolinfarbstoffe und Perylentetracarbonsäurederivate gut wirksam. Besonders gute Ergebnisse werden mit Perylen-3,4 : 9,10-tetracarbonsäurediimidderivaten erzielt, wie sie in den DE-A 31 10 954 und 31 10 960 beschrieben sind.

Für die jeweilige Verwendung kann das erfindungsgemäße elektrophotographische Aufzeichnungsmaterial übliche Zusätze enthalten, z. B. Verlaufmittel und Weichmacher in der photoleitfähigen Schicht oder Haftvermittler zwischen Träger und Schicht.

Die mit den erfindungsgemäßen Triazolylpyridinen hergestellten elektrophotographischen Aufzeichnungsmaterialien zeichnen sich durch eine Kombination sehr guter Eigenschaften, insbesondere einer hohen Photoleitfähigkeit bei gleichzeitig sehr niedriger Dunkelleitfähigkeit aus, so daß die Schichten für die Kopiertechnik sehr geeignet sind.

Deutliche Vorteile weisen diese Materialien bei der Verwendung für die Herstellung von elektrophotographischen Druckformen auf und genügen hierbei hohen Ansprüchen im Hinblick auf das Auflösungsvermögen und die Druckauflage. Die hohe Lichtempfindlichkeit erlaubt eine Senkung der Belichtungszeit bei der Verarbeitung in der Reprokamera gegenüber handelsüblichen Materialien bis etwa auf die Hälfte. Aus einer sehr randscharfen Bildwiedergabe resultiert eine gute Auflösung. Durch einen hohen Ladungskontrast können auch feine Rasterpunkte in den lichten Tonwortbereichen gut wiedergegeben werden. Ferner führt die Belichtung der Schichten zu sehr geringen Restspannungen und die bei der Betonerung erhaltenen Bilder zeichnen sich durch gute Grundfreiheit in den Nichtbildbereichen aus. Da die spektrale Empfindlichkeit der Schichten bei 600 nm stark absinkt, können die Schichten bei Rotlicht gehandhabt werden, ohne daß Bildverluste auftreten.

Die Herstellung elektrophotographischer Offsetdruckformen erfolgt dabei wie üblich durch eine elektrostatische Aufladung des elektrophotographischen Aufzeichnungsmaterials mittels einer Hochspannungscorona, eine direkt nachfolgende bildmäßige Belichtung, die Entwicklung der vorliegenden elektrostatischen, latenten Ladungsbildes mittels eines Trocken- oder Flüssigtoners, die Fixierung des Toners durch einen nachgeschalteten Schmelzvorgang und die Entfernung der unbetonerten, photohalbleitenden Schicht mittels eines geeigneten Auswaschlösemittels. Die so erhaltene Druckform kann in bekannter Weise noch für den Offsetdruck vorbereitet werden, z. B. durch eine Hydrophilierung und Gummierung der wasserführenden Oberfläche.

Die Anwendung der Triazolylpyridine der Formel (I) wird in den Anwendungsbeispielen erläutert und belegt.

Die genannten Teile und Prozentangaben beziehen sich auf das Gewicht. NMP = N-Methylpyrrolidon.

I. Herstellung der Triazolylpyridine

Beispiel 1

2-(4'-Aminophenyl)-6-diethylamino-2H-v-triazoly [4,5-b]-pyridin

a) 69 Teile p-Nitroanilin, 125 Teile Salzsäure (conc.) und 500 Teile Wasser werden bie 0 °C mit einer Lösung von 34,5 Teilen Natriumnitrit in 100 Teilen Wasser versetzt. Nach einer Stunde werden 54 Teile 2,6-Diaminopyridin in 750 Teilen Wasser zugesetzt und noch 2 Stunden mit 41 Teilen Natriumacetat abgestumpft. Der Niederschlag wird abgesaugt und mit Wasser gewaschen.

Ausbeute : 120 Teile der Verbindung der Formel (V) mit X = A = Y = H.
Schmelzpunkt : 242-245 °C.

b) Eine Lösung von 6,1 Teilen der Verbindung aus a) in 100 Teilen N-methylpyrrolidon und 50 Teilen Triethylamin werden mit 13 Teilen Kupfer-(II)-chlorid-Hydrat versetzt und 6 Stunden auf 100 °C erhitzt. Nach dem Erkalten gießt man in Wasser und saugt den Niederschlag ab.

Ausbeute : 5,5 Teile der Verbindung der Formel (VI) mit X = A = Y = H.
Schmelzpunkt : 324-328 °C.

c) 5,2 Teile der Verbindung aus b) werden in 100 Teilen Tetrahydrofuran mit 2,5 Teilen Natriumhydrid erwärmt. Nach Abklingen der Gasentwicklung werden 9,2 Teile Bromethan zugetropft und 5 Stunden gekocht. Nach Zugabe von 20 Teilen Methanol wird das Gemisch in Wasser gegossen und der Niederschlag abfiltriert.

Ausbeute : 2,5 Teile der Verbindung der (VII) mit A = X = Y = H und $R^1 = R^2 = -C_2H_5$.
Schmelzpunkt : 217-219 °C.

d) 5 Teile der nach c) hergestellten Verbindung werden in 50 Teilen Dimethylformamid bei 60 °C hydriert, als Katalysator dient Raney-Nickel. Nach dem Abkühlen wird der Katalysator abfiltriert, im Filtrat

das Amin mit Wasser gefällt, die Fällung abgesaugt und mit Wasser gewaschen.

Ausbeute : 3,6 Teile der Verbindung der Formel

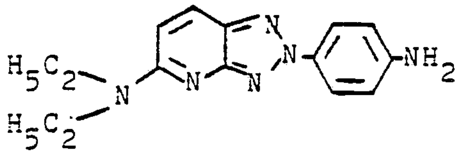

(VIIIa)

Schmelzpunkt : 144-146 °C.

Beispiel 2

8,5 Teile des nach Beispiel 1d) erhaltenen Amins werden in 75 Teilen NMP mit 20,5 Teilen Benzylbromid und 10 Teilen Natriumhydrogencarbonat 5 Stunden auf 100 °C erwärmt. Nach dem Abkühlen wird das Umsetzungsprodukt durch Zufügen von Wasser gefällt, die Fällung abgesaugt und mit Wasser gewaschen.

Zur Reinigung wurde über Kieselgel mit Essigester als Lösungsmittel chromatographiert.

Ausbeute : 6,5 Teile Verbindung (IIIa).

Schmelzpunkt : 168-170 °C.

Beispiel 3

8,5 Teile des nach Beispiel 1d) erhaltenen Amins wurden in 75 Teilen NMP mit 13,1 Teilen Ethylbromid und 10 Teilen $NaHCO_3$ 5 h bei 100 °C gerührt. Das Umsetzungsprodukt wird nach dem Abkühlen mit Wasser gefällt, die Fällung abgesaugt und mit Wasser gewaschen. Das Rohprodukt wurde aus Petrolether umkristallisiert.

Ausbeute : 5,2 Teile Verbindung (IIIb).

Schmelzpunkt : 98-100 °C.

Beispiel 4

a) 20,5 Teile der nach Beispiel 1b) erhaltenen Nitroverbindung (VI) mit X = A = Y = H wurden in 180 Teilen Tetrahydrofuran und 30 Teilen Dimethylformamid mit 9,6 Teilen Natriumhydrid und 26 Teilen Benzylbromid 5 Stunden auf Rückflußtemperatur erwärmt. Nach Zugabe von 50 Teilen Methanol wurde das Reaktionsgemisch auf Wasser ausgetragen und die Fällung abgesaugt. Nach dem Trocknen wurde aus Acetonitril umkristallisiert.

Ausbeute : 11,8 Teile Verbindung (VII) mit A = Y = X = H,

$R^1 = R^2 = -CH_2-$ 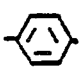

Schmelzpunkt : 121-123 °C.

b) 11,3 Teile der nach a) erhaltenen Nitroverbindung (VII) wurden in 300 Teilen Dimethylformamid in Gegenwart von Raney-Nickel bei 60 °C hydriert. Der Katalysator wurde abfiltriert und im Filtrat die Bisaminoverbindung (VIII) gefällt.

Ausbeute : 8,2 Teile Verbindung (VIII) mit A = Y = X = H und

$R^1 = R^2 = -CH_2-$ 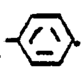

c) 8,1 Teile des nach b) erhaltenen Amins wurden in 50 Teilen NMP mit 8,7 Teilen Bromethan und 6,7 Teilen $NaHCO_3$ 5 Stunden bei 100 °C gerührt. Nach dem Abkühlen wurde das Umsetzungsprodukt mit Wasser gefällt, die Fällung abgesaugt und mit Wasser gewaschen.

Ausbeute : 2,9 Teile Verbindung (IIIc).

Schmelzpunkt : 165-167 °C.

II. Anwendung der Triazolylpyridine

Anwendungsbeispiel 1

Es wird eine 5 %ige Lösung in Tetrahydrofuran aus einer Mischung von 60 % eines Copolymeren aus

55 % Styrol, 25 % Acrylsäure, 15 % Maleinsäureanhydrid und 5 % Vinylacetat und einem K-Wert von 36, 39,5 % der Triazolylpyridinverbindung (IIIa) und 0,5 % Rhodamin 6 G (C.I. Basid Red 1, C.I. Nr. 45160) hergestellt. Diese Lösung wird nach dem Filtrieren mittels einer Rakel auf ein feingebürstetes Aluminiumblech von 105 µm Dicke so aufgetragen, daß nach dem Ablüften des Lösungsmittels und dem Trocknen (15 Min. bei 80 °C) ein Auftrag in einer Trockenschichtdicke von 4 ± 0,25 µm vorliegt. Die so erhaltene elektrophotographische Schicht wird 25 Stunden im Dunkeln gelagert. Die Schicht wird dann mittels einer Coronavorrichtung auf — 1 000 V Oberflächenpotential beladen und anschließend mit dem weißen Licht einer Xenonhochdrucklampe mit einer Beleuchtungsstärke von 0,06 mW · $cm^{-2}$ in der Bildebene belichtet. Daher fällt das Oberflächenpotential in 0,305 Sek. von 1 000 auf 500 V ab. In der gleichen Zeit fällt das Oberflächenpotential ohne Belichtung nur um 2 V auf 998 V ab.

Anwendungsbeispiel 2

Es wurde wie im Anwendungsbeispiel 1 verfahren, jedoch wurde das Copolymere durch ein Copolymer aus 76 % Styrol und 24 % Acrylsäure mit einem K-Wert von 16 ersetzt.

Die so erhaltene elektrophotographische Schicht zeigt bei Belichtung wie in Beispiel 1 einen Potentialabfall von 1 000 auf 500 V in 0,295 Sek. In der gleichen Zeit beträgt der Potentielabfall im Dunkeln 1,1 V.

Anwendungsbeispiel 3

Es wurde wie im Anwendungsbeispiel 1 verfahren, jedoch wurde anstelle der Triazolylpyridinverbindung (IIIa) die gleiche Menge der Verbindung der Formel (IIb) verwendet. Auf der so erhaltenen elektrophotographischen Schicht wurde bei Belichtung wie im Anwendungsbeispiel 1 ein Potentielabfall von 1 000 V auf 500 V in 0,315 Sek. erhalten. Im Dunkeln tritt in der gleichen Zeit ein Potentialabfall von 3,5 V auf.

Anwendungsbeispiel 4

Es wurde wie im Beispiel 1 verfahren, jedoch wurde die Triazolylpyridinverbindung (IIIa) durch die Verbindung (IIIc) ersetzt. Auf der so erhaltenen elektrophotographischen Schicht wurde beim Belichten ein Potentialabfall von 1 000 auf 500 V in 0,625 Sek. erreicht. Der Potentialabfall im Dunkeln liegt bei 8 V.

Anwendungsbeispiel 5

Auf eine Polyethylenterephthalatfolie mit einer aufgedampften, leitfähigen Aluminiumschicht in einer Dicke von etwa 300 Å wird eine Schicht aus 60 Teilen eines chlorierten Perylen-3,4 : 9,10-tetracarbonsäurediimidbisbenzimidazols mit einem Chlorgehalt von etwa 38 % und 50 Teilen eines Copolymerisates aus Vinylchlorid, Acrylsäure und einem Maleinsäurediester in einer Dicke von etwa 0,55 µm als Ladungsträger erzeugende Schicht aufgebracht.

Auf diese Ladungsträger erzeugende Schicht wird eine Ladungstransportschicht aus 55 Teilen eines handelsüblichen Bindemittels auf der Basis Polycarbonat mit einem Schmelzbereich von 220 bis 230 °C und 40 Teilen der Verbindung (IIIb) in einer Schichtdicke von 8 µm aufgebracht. Die Schicht wird mittels einer Corona auf — 1 200 V beladen und dann mit weißem Licht belichtet. Die Halbwertsphotoempfindlichkeit beträgt 250 $\mu J.cm^{-2}$.

Anwendungsbeispiel 6

50 Teile eines Copolymerisates aus 60 % Styrol und 40 % einer mit Methanol halbveresterten Maleinsäure mit einem mittleren Molekulargewicht $\bar{M}_w$ von 10.000, 40 Teile Verbindung (IIIa) und 0,2 Teile Kristallviolett (C.I. 42555) werden aus einer 5 %igen Lösung in Tetrahydrofuran auf eine elektrolytisch aufgerauhte und anodisierte Aluminiumfolie von 0,15 mm Dicke in einer Trockenschichtdicke von etwa 4 µm aufgebracht.

Diese Druckplatte wird nach einer Aufladung mittels einer Hochspannungscorona in einer Kamera bildmäßig 25 Sek. belichtet. Danach wird mit einem Pulvertoner entwickelt, der bei 160 °C abriebfest eingebrannt wird. Die unbetonerte Schicht wird mit einem Gemisch auf 5 % Soda, 25 % Isopropanol und 74,5 % Wasser abgewaschen, wodurch die Aluminiumoberfläche freigelegt wird. Die Lösungen werden mit einem Wattebausch über die Schicht gestrichen. Man erhält die im Offsetdruck erwünschte Differenzierung in hydrophile und oleophile Bereiche, wobei die Trägeroberfläche die hydrophilen Bereiche liefert.

Anschließend an die Behandung mit der alkalischen Flüssigkeit wird die Druckplatte mit Wasser nachgespült und durch Überwischen mit verbünnter Phosphorsäurelösung die Hydrophilie der Trägeroberfläche weiter erhöht. Nach Einfärben mit fetter Farbe wird auf bekannte Weise in Offsetdruckmaschinen damit gedruckt.

**Patentansprüche**

1. 2H-v-Triazolyl[4,5-b]-pyridine der Formel (I)

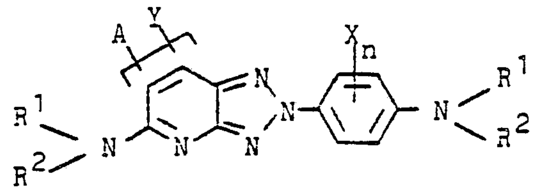

(I)

in der

$R^1$ für Wasserstoff oder wie $R^2$ für $C_1$- bis $C_4$-Alkyl, $C_5$- bis $C_7$-Cycloalkyl, für gegebenenfalls durch Chlor, Brom, $C_1$- bis $C_4$-Alkyl und/oder $C_1$- bis $C_4$-Alkoxy substituiertes Phenyl, Phenalkyl mit insgesamt 7 bis 10 C-Atomen, wobei $R^1$ und $R^2$ gleich oder verschieden sein könne, oder

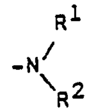

für einen gesättigten 5- oder 6-gliedrigen heterocyclischen Ring, und wobei die beiden

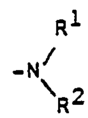

-Gruppen gleich oder verschieden sein können,

X für Wasserstoff, $C_1$- bis $C_4$-Alkyl, Halogen oder Phenyl,

n für 1 oder 2,

A für Wasserstoff, Halogen, Cyan, $C_1$- bis $C_4$-Alkyl, für gegebenenfalls durch Chlor, Brom und/oder $C_1$- bis $C_4$-Alkyl substituiertes Pheny; Phenalkyl mit insgesamt 7 bis 10 C-Atomen, $C_1$- bis $C_4$-Alkoxy, für gegebenenfalls durch Chlor, Brom und/oder $C_1$- bis $C_4$-Alkyl substituiertes Phenoxy, Phenalkoxy mit insgesamt 7 bis 10 C-Atomen oder für

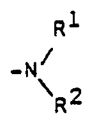

worin $R^1$ und $R^2$ oder

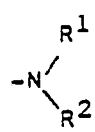

die oben angegebene Bedeutung haben, und $R^1$ auch Wasserstoff sein kann, und

Y für Wasserstoff, Halogen oder $C_1$- bis $C_{-4}$-Alkyl stehen.

2. Triazolylpyridine gemäß Anspruch 1, dadurch gekennzeichnet, daß in der Formel $R^1$ und $R^2$ für Methyl, Ethyl, Phenyl oder Benzyl oder

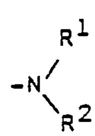

für Morpholinyl, Piperidinyl, Pyrrolidinyl oder N'-$C_1$- bis $C_4$-Alkyl-piperazinyl stehen.

3. Triazolylpyridine gemäß Anspruch 1 oder 2, dadurch gekennzeichnet, daß X für Wasserstoff steht.

4. Triazolylpyridine gemäß Anspruch 1, 2 oder 3, dadurch gekennzeichnet, daß A für Wasserstoff, Methyl, Chlor oder Cyan und Y für Methyl oder Wasserstoff stehen.

5. Triazolylpyridine gemäß Anspruch 1, gekennzeichnet durch die Formel

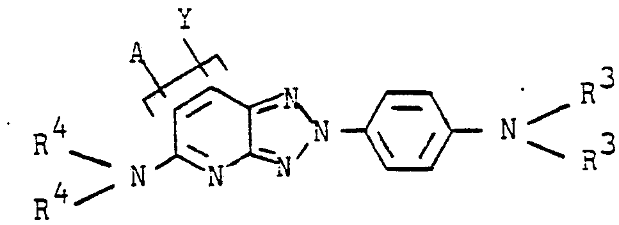

in der

$R^3$ und $R^4$ unabhängig voneinander für Methyl, Ethyl oder Benzyl,
A für Wasserstoff, Cyan, Chlor oder Methyl und
Y für Wasserstoff oder Methyl stehen.

6. Triazolylpyridine gemäß Anspruch 1, gekennzeichnet durch die Formel

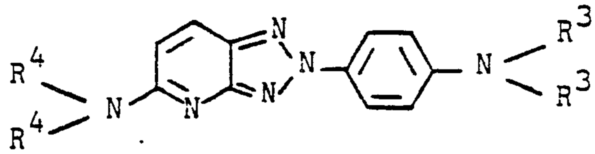

in der $—N(R^3)_2$ für

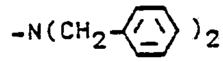

und $—N(R^4)_2$ für $—N(C_2H_5)_2$ oder $—N(R^3)_2$ und $—N(R^4)_2$ jeweils für $—N(C_2H_5)_2$ oder $—N(R^3)_2$ für $—N(C_2H_5)_2$ und $—N(R^4)_2$ für

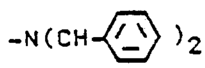

stehen.

7. Verwendung der Triazolylpyridine gemäß den Ansprüchen 1 bis 6 als Ladungsträger transportierende Verbindungen in elektrophotographischen Aufzeichnungsmaterialien.

**Claims**

1. A 2H-v-triazolol [4,5-b] pyridine of the formula (I)

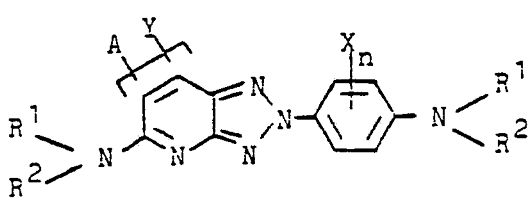 (I)

where

$R^1$ is hydrogen or, like $R^2$, is $C_1$-$C_4$-alkyl or $C_5$-$C_7$-cycloalkyl or is phenyl which is unsubstituted or substituted by chlorine, bromine, $C_1$-$C_4$-alkyl and/or $C_1$-$C_4$-alkoxy, or is phenalkyl having a total of 7 to 10 carbon atoms, and $R^1$ and $R^2$ may be identical or different, or

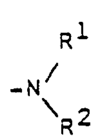

is a saturated 5-membered or 6-membered heterocyclic ring and the two

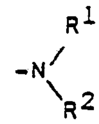

groups may be identical or different, X is hydrogen, $C_1$-$C_4$-alkyl, halogen or phenyl, n is 1 or 2, A is hydrogen, halogen, cyano or $C_1$-$C_4$-alkyl or is phenyl which is unsubstituted or substituted by chlorine, bromine and/or $C_1$-$C_4$-alkyl or is phenalkyl having a total of 7 to 10 carbon atoms or $C_1$-$C_4$-alkoxy or is phenoxy which is unsubstituted or substituted by chlorine, bromine and/or $C_1$-$C_4$-alkyl or is phenalkoxy having a total of 7 to 10 carbon atoms or is

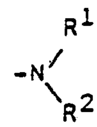

where $R^1$ and $R^2$ or

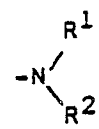

have the bove meanings, and $R^1$ may furthermore be hydrogen, and

Y is hydrogen, halogen or $C_1$-$C_4$-alkyl.

2. A triazolopyridine as claimed in claim 1, wherein, in the formula, $R^1$ and $R^2$ are each methyl, ethyl, phenyl or benzyl, or

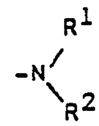

is morpholinyl, piperidinyl, pyrrolidinyl or N'-$C_1$-$C_4$-alkylpiperazinyl.

3. A triazolopyridine as claimed in claim 1 or 2, wherein X is hydrogen.

4. A triazolopyridine as claimed in claim 1, 2 or 3, wherein A is hydrogen, methyl, chlorine or cyano and Y is methyl or hydrogen.

5. A triazolopyridine as claimed in claim 1, of the formula

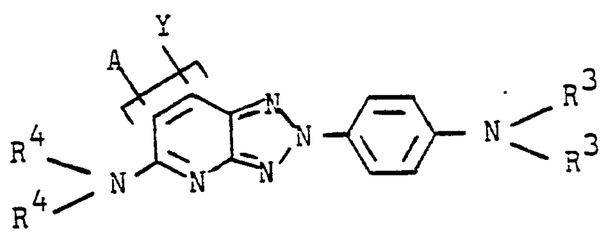

where

$R^3$ and $R^4$ independently of one another are each methyl, ethyl or benzyl,

A is hydrogen, cyano, chlorine or methyl and

Y is hydrogen or methyl.

6. A triazolopyridine as claimed in claim 1, of the formula

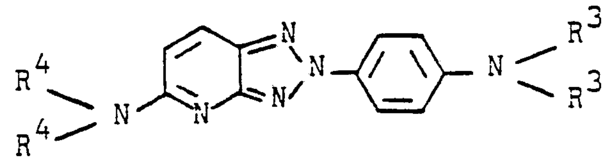

where $—N(R^3)_2$ is

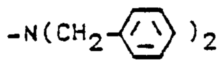

and $—N(R^4)_2$ is $—N(C_2H_5)_2$, or $—N(R^3)_2$ and $—N(R^4)_2$ are each $—N(C_2H_5)_2$, or $—N(R^3)_2$ is $—N(C_2H_5)_2$ and $—N(R^4)_2$ is

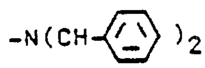

7. Use of a triazolopyridine as claimed in any of claims 1 to 6 as a charge carrier-transporting compound in electrophotographic recording materials.

**Revendications**

1. 2H-v-Triazolyl [4,5-b]-pyridines de formule

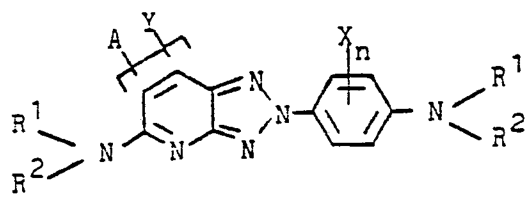

(I)

dans laquelle

$R^1$ est mis pour un atome d'hydrogène ou, de même que $R^2$, pour un radical alkyle en $C_1$ à $C_4$, cycloalkyle en $C_5$ à $C_7$, pour un radical phényle éventuellement substitué par un atome de chlore, de brome, un radical alkyle en $C_1$ à $C_4$ et/ou alcoxy en $C_1$ à $C_4$ ou pour un radical phénalkyle contenant 7 à 10 atomes de carbone au total, $R^1$ et $R^2$ pouvant être identiques ou différents, ou

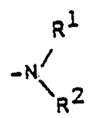

est mis pour un noyau hétérocyclique penta- ou hexagonal saturé, les deux groupements

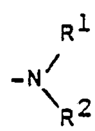

pouvant être identiques ou différents,

X est mis pour un atome d'hydrogène, un radical alkyle en $C_1$ à $C_4$, un atome d'halogène ou un groupement phényle,

n est mis pour 1 ou 2,

A est mis pour un atome d'hydrogène, d'halogène, pour un groupement cyano, alkyle en $C_1$ à $C_4$, phényle éventuellement substitué par un atome de chlore, de brome et/ou un radical alkyle en $C_1$ à $C_4$, phénalkyle contenant 7 à 10 atomes de carbone au total, alcoxy en $C_1$ à $C_4$, phénoxy éventuellement substitué par un atome de chlore, de brome et/ou un radical alkyle en $C_1$ à $C_4$, phénalcoxy contenant 7 à 10 atomes de carbone au total ou pour

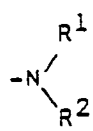

$R^1$ et $R^2$ ou

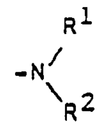

ayant les significations données ci-dessus et $R^1$ pouvant être également un atome d'hydrogène, et

Y est mis pour un atome d'hydrogène, d'halogène ou pour un radical alkyle en $C_1$ à $C_4$.

2. Triazolylpyridines selon la revendication 1, caractérisées en ce que dans la formule, $R^1$ et $R^2$ sont mis chacun pour un radical méthyle, phényle ou benzyle, ou

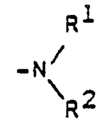

est mis pour un groupement morpholinyle, pipéridinyle, pyrrolidinyle ou N'-$C_1$ à $C_4$-alkylpipérazinyle.

3. Triazolylpyridines selon la revendication 1 ou 2, caractérisées en ce que X est mis pour un atome d'hydrogène.

4. Triazolylpyridines selon l'une quelconque des revendications 1 à 3, caractérisées en ce que A est mis pour un atome d'hydrogène, un groupement méthyle, un atome de chlore ou un groupement cyano et Y pour un radical méthyle ou un atome d'hydrogène.

5. Triazolylpyridines selon la revendication 1, caractérisées par la formule

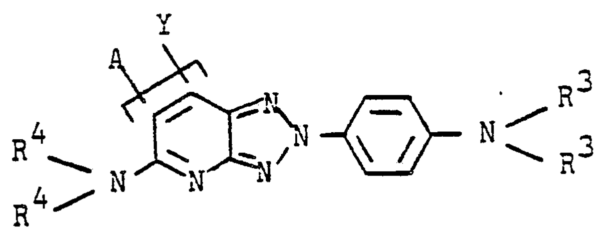

dans laquelle

$R^3$ et $R^4$, indépendamment l'un de l'autre, sont mis chacun pour un radical méthyle, éthyle ou benzyle.

A est mis pour un atome d'hydrogène, un groupement cyano, un atome de chlore ou un groupement méthyle et

Y pour un atome d'hydrogène ou un radical méthyle.

6. Triazolylpyridines selon la revendication 1, caractérisées par la formule

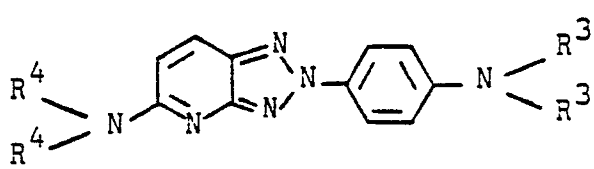

dans laquelle $-N(R^3)_2$ est mis pour

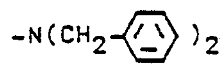

et $-N(R^4)_2$ pour $-N(C_2H_5)_2$, ou $-N(R^3)_2$ et $-N(R^4)_2$ sont mis chacun pour $-N(C_2H_5)_2$, ou $-N(R^3)_2$ est mis pour $-N(C_2H_5)_2$ et $-N(R^4)_2$ pour

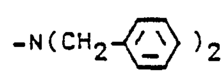

7. Utilisation des triazolylpyridines selon l'une quelconque des revendications 1 à 6 comme composés transporteurs de porteurs de charge dans des matériels d'enregistrement électrophotographique.